# EUROPEAN PATENT SPECIFICATION

(11) **EP 4 003 242 B1**
(45) Date of publication and mention of the grant of the patent: **31.05.2023**
(21) Application number: 20767598.4
(22) Date of filing: 28.07.2020
(51) Int. Cl.: A61F 2/95, A61F 2/94, A61F 2/848, A61M 27/00, A61F 2/04, A61F 2/82

(54) **STENT AND CORRESPONDING INSERTION DEVICE**
STENT UND ENTSPRECHENDE EINFÜHRVORRICHTUNG
ENDOPROTHÈSE ET DISPOSITIF D'INSERTION CORRESPONDANT

(30) Priority: 29.07.2019 IT 201900013206
(43) Date of publication of application: 01.06.2022
(73) Proprietor: Euromedical S.R.L., 25010 San Zeno Naviglio (BS) (IT)
(72) Inventor: BONERA, Ilario, 25121 Brescia (IT); RAMORINO, Giorgio, 25010 San Zeno Naviglio (BS) (IT)
(74) Representative: Cernuzzi, Daniele
(86) International application number: PCT/IB2020/057096
(87) International publication number: WO 2021/019432

(56) References cited:
- US-A1- 2001 021 835
- US-A1- 2005 228 481
- US-A1- 2005 283 228
- US-A1- 2011 190 865

## Description

### TECHNICAL FIELD

The present invention relates to a stent and a device for inserting such stent.

### BACKGROUND ART

The term stent generally refers to an expandable prosthesis which is introduced in a body duct or lumen organ to restore its patency when this is reduced or impaired.

Stents, for example, are used in blood vessels (arteries and veins) and in other tubular structures such as the bile duct, oesophagus, trachea, ureter, etcetera.

The stent is inserted into the duct to be treated, normally by a catheter, in a minimum size retracted form; once in position, the stent is expanded and takes on a substantially tubular expanded shape of use.

Despite various types of stents and corresponding insertion devices are known, there still seems to be room for improvement in the prior art stents and the corresponding insertion devices.

In particular, various types of stents are made of metal materials, possibly shape memory materials, and are thus particularly complicated and expensive to make, even generally having relatively great sizes especially if wide dilations are required.

The use of different materials, in particular polymeric materials, has not been, for the time being, tangibly applied.

Furthermore, the prior art stents cannot normally be progressively expanded and it is not possible to decide the degree of the stent dilation during the implantation step.

An example of such stents is disclosed in US 2001/021835.

Further, the known stents cannot normally be recovered, namely once having been dilated they can no longer be retracted and moved, for instance for correcting a possible imprecision in the implantation position.

### DISCLOSURE OF INVENTION

It is an object of the present invention to provide a stent and a corresponding operating device for insertion thereof that overcome the above noted drawbacks of the prior art.

The present invention thus relates to a stent and a device for inserting a stent as defined in the enclosed claims 1 and, respectively, 7.

Preferred auxiliary features are defined in the dependent claims.

The stent of the invention is easy to make and use as well as fully efficient and reliable.

The stent is preferably made of polymeric material (plastic), thus being of particularly easy and cheap construction.

Furthermore, the stent of the invention has a progressive dilation and is progressively dilated remaining stable in each configuration taken on: it is thus possible to decide the degree of dilation of the stent while implanting it.

The stent can also be recovered, as it has a reversible dilation: once having been dilated, the stent can always be taken back to the initial retracted configuration, for instance to be repositioned.

In particular, the stent can be recovered in a sectional way, allowing to select the number of recover sections.

The stent can also be provided with one or more radiopaque markers, at a distal, proximal position and/or at an intermediate position between the distal and proximal position.

### BRIEF DESCRIPTION OF THE DRAWINGS

Further features and advantages of the present invention will become clear from the description of the following non-limiting embodiments, with reference to the figures of the accompanying drawings, wherein:
- figure 1 is a perspective schematic, partially sectioned view of a stent and corresponding insertion device according to the invention;
- figure 2 is a longitudinal partially sectioned view of the device of figure 1;
- figure 3 is an exploded perspective schematic view of the device of figure 1;
- figure 4 is an enlarged scale perspective view of the stent according to the invention;
- figure 5 is an enlarged scale perspective view of a component of figure 1;
- figures 6 and 7 are enlarged scale respective views of the details highlighted in figure 2;
- figures 8-18 schematically show the functioning of the device of the invention, represented in a side view in several positions of use (figures 8-9, 11, 13, 15, 17-18) and by certain enlarged scale and longitudinal sectioned details (figures 10, 12, 14, 16).

### BEST MODE FOR CARRYING OUT THE INVENTION

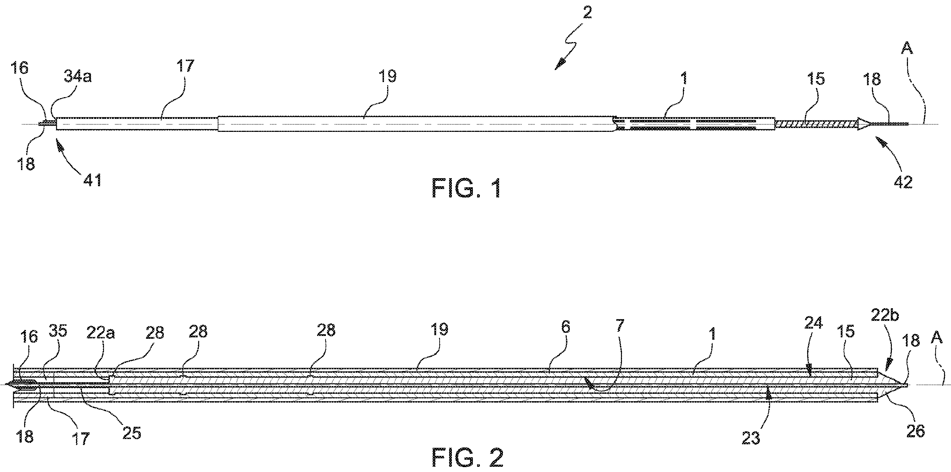

In figures 1-3 a stent 1 is shown together with an insertion device 2 for inserting the stent 1 in a body duct or lumen organ.

The stent 1 is illustrated in a minimum size closed configuration, to be used while inserting and positioning the stent 1 by the device 2.

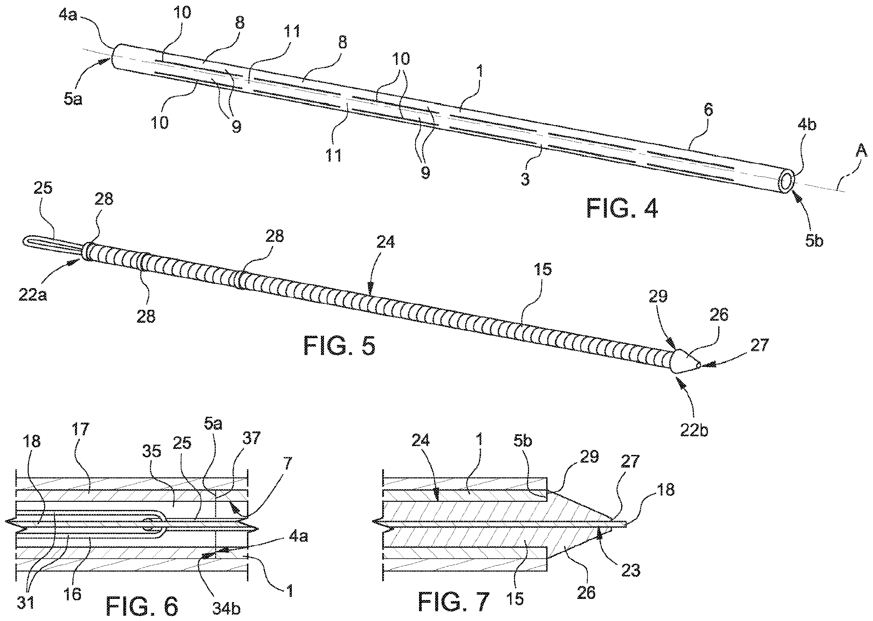

Referring specifically also to figure 4, the stent 1 has a hollow tubular body 3 extending along and about a longitudinal axis A and for example substantially cylindrical.

According to a preferred aspect of the invention, the body 3 of the stent 1 is made of polymeric material (plastic), such as a polymeric elastomer material (such as, though not necessarily, selected among silicones, polyurethanes, TPE and similar) or a thermo-plastic polymeric material belonging to the biomedical polymeric category (such as, though not necessarily polyamide, acetalic resin, polypropylene, polyethylene, etcetera).

The body 3 extends between two opposite open axial ends 4a, 4b, provided with respective annular end edges 5a, 5b, and has a substantially cylindrical lateral wall 6 delimiting a longitudinal inner conduit 7 extending between the ends 4a, 4b.

The body 3 comprises a plurality of deformable sections 8 arranged in succession along the axis A.

Each section 8 comprises a plurality of longitudinal portions 9 of the lateral wall 6 and a plurality of radial cuts 10 formed through the lateral wall 6 and separating the longitudinal portions 9 from one another.

The cuts 10 of each section 8 are in particular substantially parallel to one another and angularly staggered about the axis A.

The cuts 10 are for instance in the form of slots longitudinally elongated parallel to the axis A.

Each section 8 has two or more cuts 10 and is axially delimited by a pair of annular portions 11 of the lateral wall 6.

The annular portions 11 of the lateral wall 6 thus join to one another the sections 8 and separate the cuts 10 of the various sections 8 that axially succeed on the body 3.

In the illustrated non-limiting example, though not necessarily, the cuts 10 of the various sections 8 are aligned between each other and have a similar length. It must be understood that the cuts 10 can be differently arranged and organised and be placed at a different pitch and/or orientation from what herein disclosed and illustrated for merely exemplary purposes.

Referring again to figures 1-3, the device 2 for inserting the stent 1 extends along the axis A and comprises: an operating member 15, an actuation wire 16, a pusher 17, a guide wire 18 and optionally a cover sheath 19.

The operating member 15, illustrated in greater detail in figure 5, has an internally hollow flexible tubular body, made for instance (though not necessarily) of metal material. For instance, the operating member 15 has a corrugated or spiral structure tubular body.

The operating member 15 extends longitudinally along the axis A between opposite open longitudinal ends 22a, 22b and has an inner longitudinal conduit 23 passing entirely through the operating member 15 between the ends 22a, 22b.

The operating member 15 has an outer lateral surface 24, optionally (depending also on the constructive structure of the operating member 15) wavy or coiled.

The end 22a is provided with an eyelet 25 projecting from the end 22a along the axis A; the opposite end 22b is provided with a pilot head 26, radially projecting from the surface 24 and staggered towards the end 22b or substantially conical or truncated-cone. The head 26 ends with a front opening 27 in communication with the conduit 23.

The surface 24 has a series of annular projections 28, radially projecting from the surface 24 and longitudinally spaced apart from one another along the operating member 15. In particular, the operating member 15 has a first projection 28 at the end 22a and further successive projections 28 along the axis A.

Projections 28 extend from the surface 24 to contact an inner lateral surface of the stent 1, when the operating member 15 is housed in the conduit 7 of the stent 1, or of the pusher 17, when the operating member 15 slides outside the conduit 7 of the stent 1 inside the pusher 17, as will be clearly explained hereinafter.

In the device 2 ready for use, i.e. for introducing the stent 1 in a patient (figure 1), the operating member 15 in arranged inside the stent 1 and in particular inside the conduit 7 of the stent 1, i.e. the stent 1 is fitted about the operating member 15 and surrounds it radially outside.

The eyelet 25 and the head 26 project axially, at least partially, outside the conduit 7 from respective ends 4a, 4b of the stent 1.

In particular, the eyelet 25 projects axially, at least partially, from the end 4a beyond the edge 5a (figure 6); and the head 26 projects from the end 4b beyond the edge 5b and axially abuts against the edge 5b with an annular shoulder 29 facing the edge 5b of the stent 1 (figure 7).

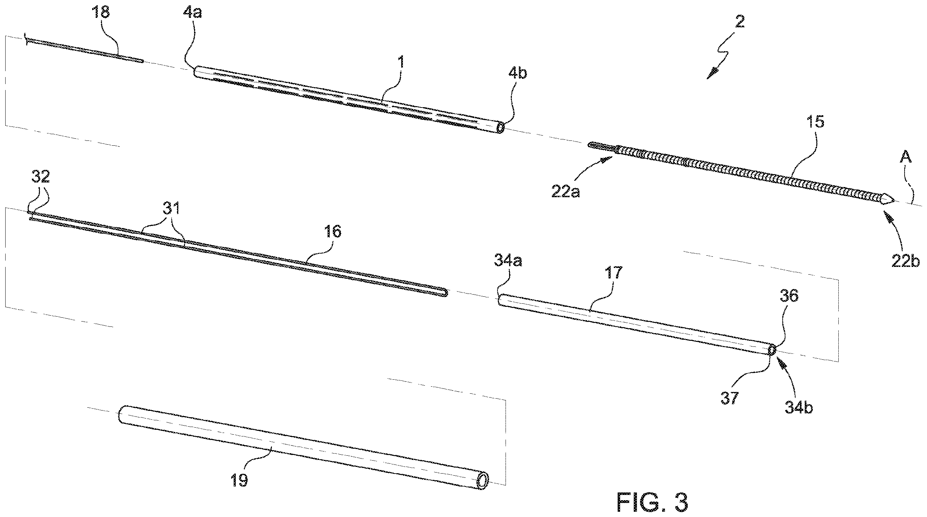

Referring specifically to figures 3 and 6, the actuation wire 16 is a wire, such as a metal wire, that is U-folded so as engage the eyelet 25 of the operating member 15; in particular, the actuation wire 16 is inserted into the eyelet 26 and U-folded so as to have a pair of parallel arms 31 exiting from the eyelet 25 and ending with respective ends 32.

Referring specifically to figures 3 and 6, the pusher 17 is a tubular element, for instance of metal or polymeric material (plastic), extending between two opposite open axial ends 34a, 34b.

The pusher 17 is provided with an inner longitudinal conduit 35 extending from the ends 34a, 34b.

The pusher 17 is axially aligned to the stent 1 along the axis A and has a front edge 36, placed at the end 34b, defining an axial shoulder 37 axially abutting against the edge 5a of the stent 1.

The guide wire 18 is for guiding the insertion of the device 2 into the patient's organ where the stent 1 must be implanted; for instance, the guide wire 18 is a metal wire capable of transmitting axial forces (along a wire longitudinal axis) in opposite directions.

The guide wire 18 passes through the entire device 2 along the axis A: in particular, the guide wire 18 is inserted in the conduit 23 of the operating member 15 (axially projecting from the opposite ends 22a, 22b of the operating member 15), in the conduit 7 of the stent 1, and in the conduit 35 of the pusher 17; the guide wire 18 projects outside from the end 34a of the pusher 17 with the arms 31 of the actuation wire 16; and from the end 22b of the operating member 15 through the opening 27.

The (optional) cover sheath 19, for example made of polymeric material, is placed about the stent 1 and at least a portion of the pusher 17.

Once the device 2 is assembled (figure 1) the device 2 extends along the axis A between a proximal end 41, defined by the end 34a of the pusher 17 and from which a first end of the guide wire 18 and two arms 31 placed side by side of the actuation wire 16 project outside; and a distal end 42, defined by the end 22b of the actuating member 15 and from which a second end of the guide wire 18 projects outside.

The stent 1 is implanted by of the device 2, which can be operated by any instrument of the known type for this kind of operations, which does not pertain to the present invention and not herein described nor illustrated for ease of simplicity.

Once the stent 1 is mounted on the device 2, in the assembled configuration shown in figure 1, the device 2 is introduced in the hollow organ where the stent 1 must be implanted.

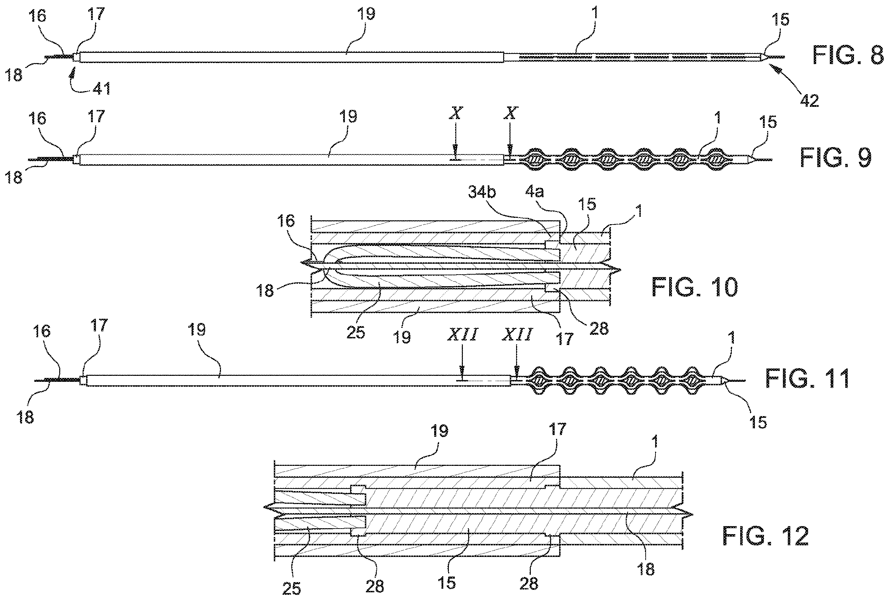

Once in position, the stent 1 is extracted from the cover sheath 19 (if this is present) acting on the pusher 17 at the proximal end 41 of the device 2 (figure 8). In particular, the pusher 17 is moved in an axial direction along the axis A towards the distal end 42 of the device 2; the shoulder 37 of the pusher 17 pushes the edge 5a of the stent 1 and thus pushes the stent 1 out of the cover sheath 19.

The stent 1 is thus radially dilated (figure 9) acting on the actuation wire 16, which is pulled backward, i.e. moved away from the proximal end 41 of the device 2, while the pusher 17 is maintained axially fixed: the actuation wire 16, being hooked to the eyelet 25, moves the operating member 15 backward acting on the end 4b of the stent 1, by the head 26 cooperating with the edge 5b of the stent 1.

The end 4b of the stent 1 moves back, upon being pushed by the shoulder 29 of the head 26 of the operating member 15; as the opposite end 4a is axially blocked by the edge 36 (by the shoulder 37) of the pusher 17 contacting the edge 5a, the stent 1 shortens along the axis A and deforms radially, thanks to the cuts 9 that allow the deformation of the sections 8 and in particular of the portions 9.

The axial squeezing of the stent 1 (i.e. the movement of the opposite ends 4a, 4b thereof close to one another)thereby causes the radial expansion of the sections 8 which take on an arched shape. The stent 1 takes on a wavy conformation with a number of waves defined by the sections 8.

The sections 8 are plastically deformable, i.e. they stay in the deformed shape taken on upon being stressed (in particular, after axial compression of the body 3 of the stent 1) even after been stressed; the overall sections 8 and stent 1 return to the initial position (i.e. in the minimum size closed configuration of the stent 1) if submitted to a contrary stress, namely if the body 3 of the stent 1 is submitted to elongation, moving the ends 4a, 4b away from each other).

The operating member 15 is axially moved relative to the pusher 17, which remains fixed; the axial position along the axis A of the operating member 15 relative to the pusher 17 can be adjusted by projections 28 which the operating member 15 is provided with.

In fact (figure 10), when a projection 28 exits from the end 4a of the stent 1 and contacts the pusher 17, for instance engaging an annular seat formed on an inner lateral surface of the pusher 17 at the end 34b of the pusher 17, the user operating the device 2 perceives the position snap of the operating member 15 and knows where the operating member 15 is in relation to the stent 1 as well as the dilation degree of the stent 1.

Continuing the movement of the operating member 15 to the subsequent projection 28 the stent 1 continues to deform dilating progressively (figures 11-12).

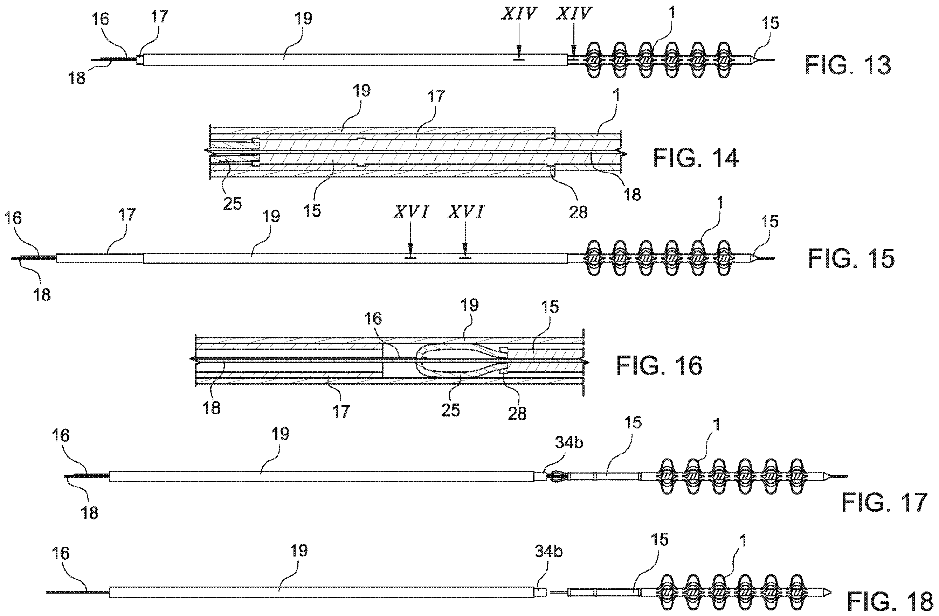

When the last projection 28 exits from the stent 1, the stent 1 is completely expanded (figures 13-14).

Now, the pusher 17 is moved backward along the axis A (i.e. it is moved away from the distal end 42 of the device 2) and is moved out of the operating member 15 (figures 15-16). The eyelet 25 of the operating member 15 exits from the pusher 17 and it is free to deform taking on the size allowed by the cover sheath 19.

The pusher 17 is then again moved forward along the axis A, i.e. towards the distal end 42 of the device 2, until it projects outside with its end 34b from the cover sheath 19 and thus also pushes the operating member 15 out of the cover sheath 19(figure 17).

Removal is carried out of the guide wire 18, which is simply pulled backwards and moved out of the conduit 7 of the stent 1, from the conduit 23 of the operating member 15, and from the conduit 35 of the pusher 17; and of the actuation wire 16, which is extracted pulling one of the ends 32 and keeping the other end 32 free, in order to clear the eyelet 25 (figure 18).

By contrast the operating member 15 remains inside the stent 1.

## Claims

1. A stent (1) comprising a hollow tubular body (3) extending along and about a longitudinal axis (A) between two opposite open axial ends (4a, 4b) and having a substantially cylindrical lateral wall (6) delimiting a longitudinal inner conduit (7); the body (3) comprising a plurality of deformable sections (8) arranged in succession along the axis (A); each section (8) comprising a plurality of longitudinal portions (9) of the lateral wall (6) and a plurality of radial cuts (10) formed through the lateral wall (6) and which separate the longitudinal portions (9) from one another; **characterized in that** the sections (8) are plastically deformable as a consequence of axial compression and expansion of the body (3) remaining in a deformed shape taken on upon being stressed after axial compression of the body (3) even after been stressed, and returning to a minimum size closed configuration if the body (3) is submitted to elongation, so that the stent (1) has a progressive dilation and can be progressively dilated remaining stable in each configuration taken on and the stent (1) has a reversible dilation and once having been dilated can be taken back to the initial retracted configuration.

2. A stent according to claim 1, wherein the cuts (10) of each section (8) are substantially parallel to one another and angularly staggered about the axis (A) with respect to one another.

3. A stent according to claim 1 or 2, wherein the cuts (10) are in the form of slots longitudinally elongated parallel to the axis (A).

4. A stent according to one of the preceding claims, wherein each section (8) has two or more cuts (10) and is axially delimited by a pair of annular portions (11) of the lateral wall (6) which join to one another the sections (8) and separate the cuts (10) of the respective sections (8).

5. A stent according to one of the preceding claims, wherein the stent (1) is made of polymer material.

6. An insertion device (2) for inserting a stent (1) according to one of the preceding claims in a body duct or lumen organ; the device (2) extending along the axis (A) between a proximal end (41) and a distal end (42) and comprising: said stent (1); and an operating member (15) and a pusher (17), having respective opposite axial shoulders (29, 37) axially cooperating in contact with respective opposite axial ends (4a, 4b) of the stent (1) and movable with respect to one another along the axis (A) to axially compress the stent (1) and cause deformation of the deformable sections (8) of the stent (1).

7. A device according to claim 6, wherein the pusher (17) is defined by a tubular element axially aligned to the stent (1) along the axis (A) outside the stent (1) and cooperates with a first end (4a) of the stent (1) via a first shoulder (37) defined by an annular front edge (36) of the pusher (17); and wherein the operating member (15) is housed inside the conduit (7) of the stent (1) and projects outside a second end (4b) of the stent with a head (26) provided with a second shoulder (29) facing and cooperating with said second end (4b) of the stent (1).

8. A device according to claim 6 or 7, comprising an actuation wire (16) engaging an eyelet (25) of the operating member (15) to axially move the operating member (15) towards the proximal end (41) of the device (2).

9. A device according to claim 8, wherein the actuation wire (16) is inserted in the eyelet (26) and U-folded so as to have a pair of parallel arms (31) exiting from the eyelet (25) and ending with respective ends (32).

10. A device according to one of claims 6 to 9, wherein the operating member (15) has a flexible internally hollow tubular body.

11. A device according to one of claims 6 to 10, wherein the operating member (15) is provided with a series of annular projections (28), radially projecting from an outer lateral surface (24) of the operating member (15) and longitudinally spaced apart from one another along the operating member (15) to contact radially an inner lateral surface of the stent (1), when the operating member (15) is housed in the conduit (7) of the stent (1), or of the pusher (17), when the operating member (15) slides outside the conduit (7) of the stent (1) and inside the pusher (17).

12. A device according to one of claims 6 to 11, wherein the operating member (15) has a corrugated or spiral structure tubular body, preferably made of metal material.

13. A device according to one of claims 6 to 12, comprising a guide wire (18) arranged along the axis (A) through the device (2) and exiting from said proximal end (41) and from said distal end (42) of the device (2); the guide wire (18) being housed in respective conduits (7, 23, 35) formed through the stent (1), the operating member (15) and the pusher (17).

## Patentansprüche

1. Stent (1), welcher einen hohlen rohrförmigen Körper (3) umfasst, der sich entlang einer und um eine Längsachse (A) zwischen zwei entgegengesetzten offenen axialen Enden (4a, 4b) erstreckt und eine im Wesentlichen zylindrische Seitenwand (6) aufweist, die einen inneren Längskanal (7) begrenzt; wobei der Körper (3) mehrere verformbare Teilstücke (8) umfasst, die hintereinander entlang der Achse (A) angeordnet sind; wobei jedes Teilstück (8) mehrere Längsabschnitte (9) der Seitenwand (6) und mehrere radiale Einschnitte (10), die durch die Seitenwand (6) hindurch ausgebildet sind und die Längsabschnitte (9) voneinander trennen, umfasst; **dadurch gekennzeichnet, dass** die Teilstücke (8) infolge einer axialen Kompression und Ausdehnung des Körpers (3) plastisch verformbar sind, wobei sie in einer verformten Gestalt, die angenommen wird, wenn sie nach axialer Kompression des Körpers (3) beansprucht werden, auch verbleiben, nachdem sie beansprucht wurden, und wobei sie zu einer geschlossenen Konfiguration minimaler Größe zurückkehren, falls der Körper (3) einer Streckung unterzogen wird, so dass der Stent (1) eine zunehmende Dehnung aufweist und zunehmend gedehnt werden kann, wobei er in jeder angenommenen Konfiguration stabil bleibt, und der Stent (1) eine reversible Dehnung aufweist und, nachdem er gedehnt worden ist, in die ursprüngliche zurückgezogene Konfiguration zurückversetzt werden kann.

2. Stent nach Anspruch 1, wobei die Einschnitte (10) jedes Teilstücks (8) im Wesentlichen parallel zueinander und um die Achse (A) zueinander winkelmäßig versetzt sind.

3. Stent nach Anspruch 1 oder 2, wobei die Einschnitte (10) in der Form von Schlitzen vorliegen, die sich parallel zur Achse (A) in Längsrichtung erstrecken.

4. Stent nach einem der vorhergehenden Ansprüche, wobei jedes Teilstück (8) zwei oder mehr Einschnitte (10) aufweist und axial von einem Paar ringförmiger Abschnitte (11) der Seitenwand (6) begrenzt wird, welche die Teilstücke (8) miteinander verbinden und die Einschnitte (10) der jeweiligen Teilstücke (8) trennen.

5. Stent nach einem der vorhergehenden Ansprüche, wobei der Stent (1) aus Polymermaterial hergestellt ist.

6. Einführvorrichtung (2) zum Einführen eines Stents (1) nach einem der vorhergehenden Ansprüche in einen Körpergang oder ein Lumenorgan; wobei sich die Vorrichtung (2) entlang der Achse (A) zwischen einem proximalen Ende (41) und einem distalen Ende (42) erstreckt und umfasst: den Stent (1); und ein Betätigungselement (15) und einen Schieber (17), die jeweilige entgegengesetzte axiale Schultern (29, 37) aufweisen, die axial in Kontakt mit jeweiligen entgegengesetzten axialen Enden (4a, 4b) des Stents (1) zusammenwirken und relativ zueinander entlang der Achse (A) beweglich sind, um den Stent (1) axial zusammenzudrücken und eine Verformung der verformbaren Teilstücke (8) des Stents (1) zu bewirken.

7. Vorrichtung nach Anspruch 6, wobei der Schieber (17) durch ein rohrförmiges Element definiert ist, das zu dem Stent (1) außerhalb des Stents (1) entlang der Achse (A) axial ausgerichtet ist und mit einem ersten Ende (4a) des Stents (1) über eine erste Schulter (37) zusammenwirkt, die durch einen ringförmigen vorderen Rand (36) des Schiebers (17) definiert ist; und wobei das Betätigungselement (15) im Inneren des Kanals (7) des Stents (1) untergebracht ist und aus einem zweiten Ende (4b) des Stents mit einem Kopf (26) herausragt, der mit einer zweiten Schulter (29) versehen ist, die dem zweiten Ende (4b) des Stents (1) zugewandt ist und mit ihm zusammenwirkt.

8. Vorrichtung nach Anspruch 6 oder 7, welche einen Betätigungsdraht (16) umfasst, der in eine Öse (25) des Betätigungselements (15) eingreift, um das Betätigungselement (15) axial in Richtung des proximalen Endes (41) der Vorrichtung (2) zu bewegen.

9. Vorrichtung nach Anspruch 8, wobei der Betätigungsdraht (16) in die Öse (26) eingeführt und U-förmig umgebogen ist, so dass er ein Paar paralleler Arme (31) aufweist, die aus der Öse (25) austreten und mit entsprechenden Enden (32) enden.

10. Vorrichtung nach einem der Ansprüche 6 bis 9, wobei das Betätigungselement (15) einen flexiblen, innen hohlen rohrförmigen Körper aufweist.

11. Vorrichtung nach einem der Ansprüche 6 bis 10, wobei das Betätigungselement (15) mit einer Reihe von ringförmigen Vorsprüngen (28) versehen ist, die von einer äußeren Seitenfläche (24) des Betätigungselements (15) radial vorstehen und entlang des Betätigungselements (15) in Längsrichtung voneinander beabstandet sind, um radial mit einer inneren Seitenfläche des Stents (1), wenn das Betätigungselement (15) im Kanal (7) des Stents (1) untergebracht ist, oder des Schiebers (17), wenn das Betätigungselement (15) außerhalb des Kanals (7) des Stents (1) und innerhalb des Schiebers (17) gleitet, in Kontakt zu kommen.

12. Vorrichtung nach einem der Ansprüche 6 bis 11, wobei das Betätigungselement (15) einen rohrförmigen Körper mit gerippter oder spiralförmiger Struktur aufweist, der vorzugsweise aus Metallmaterial hergestellt ist.

13. Vorrichtung nach einem der Ansprüche 6 bis 12, welche einen Führungsdraht (18) umfasst, der entlang der Achse (A) durch die Vorrichtung (2) hindurch angeordnet ist und aus dem proximalen Ende (41) und aus dem distalen Ende (42) der Vorrichtung (2) austritt; wobei der Führungsdraht (18) in jeweiligen Kanälen (7, 23, 35) untergebracht ist, die durch den Stent (1), das Betätigungselement (15) und den Schieber (17) hindurch ausgebildet sind.

## Revendications

1. Endoprothèse (1) comprenant un corps tubulaire creux (3) s'étendant le long et autour d'un axe longitudinal (A) entre deux extrémités axiales ouvertes opposées (4a, 4b) et ayant une paroi latérale sensiblement cylindrique (6) délimitant un conduit interne longitudinal (7) ; le corps (3) comprenant une pluralité de sections déformables (8) disposées successivement le long de l'axe (A) ; chaque section (8) comprenant une pluralité de parties longitudinales (9) de la paroi latérale (6) et une pluralité de découpes radiales (10) formées à travers la paroi latérale (6) et qui séparent les parties longitudinales (9) les unes des autres ; **caractérisée en ce que** les sections (8) sont plastiquement déformables suite à une compression axiale et à une expansion du corps (3) restant dans un état déformé adopté sous contrainte suivant une compression axiale du corps (3) même après mise sous contrainte, et revenant à une configuration fermée de taille minimale si le corps (3) est soumis à une élongation, de sorte que l'endoprothèse (1) a une dilatation progressive et peut être progressivement dilatée en restant stable dans chaque configuration adoptée et que l'endoprothèse (1) a une dilatation réversible et peut, après avoir été dilatée, être ramenée à la configuration initiale rétractée.

2. Endoprothèse selon la revendication 1, dans laquelle les découpes (10) de chaque section (8) sont sensiblement parallèles les unes aux autres et sont réparties angulairement les unes par rapport aux autres autour de l'axe (A).

3. Endoprothèse selon la revendication 1 ou 2, dans laquelle les découpes (10) ont la forme de fentes longitudinalement allongées, parallèles à l'axe (A).

4. Endoprothèse selon l'une des revendications précédentes, dans laquelle chaque section (8) a deux découpes (10) ou plus et est axialement délimitée par une paire de parties annulaires (11) de la paroi latérale (6) qui relient entre elles les sections (8) et séparent les découpes (10) des sections (8) respectives.

5. Endoprothèse selon l'une des revendications précédentes, dans laquelle l'endoprothèse (1) est en matériau polymère.

6. Dispositif d'insertion (2) pour insérer une endoprothèse (1) selon l'une des revendications précédentes dans un canal corporel ou une lumière d'un organe ; le dispositif (2) s'étendant le long de l'axe (A) entre une extrémité proximale (41) et une extrémité distale (42) et comprenant : ladite endoprothèse (1) ; et un élément de commande (15) et un poussoir (17), ayant des épaulements axiaux opposés respectifs (29, 37) coopérant axialement en contact avec des extrémités axiales opposées respectives (4a, 4b) de l'endoprothèse (1) et mobiles l'un par rapport à l'autre le long de l'axe (A) pour comprimer axialement l'endoprothèse (1) et entraîner une déformation des sections déformables (8) de l'endoprothèse (1).

7. Dispositif selon la revendication 6, dans lequel le poussoir (17) est délimité par un élément tubulaire axialement aligné avec l'endoprothèse (1) le long de l'axe (A) à l'extérieur de l'endoprothèse (1) et coopère avec une première extrémité (4a) de l'endoprothèse (1) par l'intermédiaire d'un premier épaulement (37) défini par un bord avant annulaire (36) du poussoir (17) ; et dans lequel l'élément de commande (15) est logé à l'intérieur du conduit (7) de l'endoprothèse (1) et fait saillie à l'extérieur d'une seconde extrémité (4b) de l'endoprothèse avec une tête (26) pourvue d'un second épaulement (29) faisant face et coopérant avec ladite seconde extrémité (4b) de l'endoprothèse (1).

8. Dispositif selon la revendication 6 ou 7, comprenant un fil d'actionnement (16) engageant un oeillet (25) de l'élément de commande (15) pour déplacer axialement l'élément de commande (15) vers l'extrémité proximale (41) du dispositif (2).

9. Dispositif selon la revendication 8, dans lequel le fil d'actionnement (16) est inséré dans l'oeillet (26) et plié en U de manière à ce qu'une paire de branches parallèles (31) sortent de l'oeillet (25) et se terminent par des extrémités respectives (32).

10. Dispositif selon l'une des revendications 6 à 9, dans lequel l'élément de commande (15) a un corps tubulaire flexible creux à l'intérieur.

11. Dispositif selon l'une des revendications 6 à 10, dans lequel l'élément de commande (15) est pourvu d'une série de protubérances annulaires (28), faisant saillie radialement depuis une surface latérale externe (24) de l'élément de commande (15) et espacées longitudinalement les unes des autres le long de l'élément de commande (15) pour venir en contact radialement avec une surface latérale interne de l'endoprothèse (1), lorsque l'élément de commande (15) est logé dans le conduit (7) de l'endoprothèse (1), ou du poussoir (17), lorsque l'élément de commande (15) glisse à l'extérieur du conduit (7) de l'endoprothèse (1) et à l'intérieur du poussoir (17).

12. Dispositif selon l'une des revendications 6 à 11, dans lequel l'élément de commande (15) a un corps tubulaire à structure ondulée ou en spirale, de préférence en matériau métallique.

13. Dispositif selon l'une des revendications 6 à 12, comprenant un fil de guidage (18) disposé le long de l'axe (A) à travers le dispositif (2) et sortant de ladite extrémité proximale (41) et de ladite extrémité distale (42) du dispositif (2) ; le fil de guidage (18) est logé dans des conduits respectifs (7, 23, 35) formés à travers l'endoprothèse (1), l'élément de commande (15) et le poussoir (17).
